# EUROPEAN PATENT SPECIFICATION

(11) **EP 0 662 006 B1**
(45) Date of publication and mention of the grant of the patent: **24.11.1999**
(21) Application number: 93920455.8
(22) Date of filing: 01.09.1993
(51) Int. Cl.: A61L 31/00, A61F 9/00, A61F 6/22, A61B 17/12, A61B 17/00

(54) **Polymer compositions and device for OCCLUSION OF CHANNELS IN LIVING MAMMALS**
Polymerzusammensetzungen und Apparat zum VERSCHLIESSEN VON KANÄLEN IN LEBENDEN SÄUGETIEREN
Compositions de polymères et appareil pour l' OCCLUSION DE CANAUX CHEZ LES MAMMIFERES VIVANTS

(30) Priority: 02.09.1992 US 939110
(43) Date of publication of application: 12.07.1995
(73) Proprietor: LANDEC CORPORATION, Menlo Park, CA 94025 (US)
(72) Inventor: SCHMITT, Edward, E., Palo Alto, CA 94306 (US)
(74) Representative: Hall, Robert Leonard
(86) International application number: PCT/US93/08245
(87) International publication number: WO 94/05342

(56) References cited:
- WO-A-81/00701
- WO-A-90/13420
- GB-A- 2 223 025
- US-A- 3 422 813

## Description

This invention relates to compositions and apparatus suitable for occluding channels in living mammals.

It is known to occlude certain channels in living mammals, for example, tear ducts to relieve keratoconjunctivitis sicca or KCS (commonly known as dry eye syndrome), fallopian tubes to prevent pregnancy, blood vessels to close off blood supply, and bone cavities to support pins. For example, preformed plugs and collagen implants have been used to occlude tear ducts (see U.S. Patent Nos. 3,949,750 and 4,959,048); and plugs formed in situ from polymerizable monomers have been used in various situations, including plugs formed from thermosetting silicones to occlude fallopian tubes, from cyanoacrylate monomers to seal blood vessels, and from methyl methacrylate monomers to fill bone cavities. These known methods suffer from serious problems. For example, collagen implants dissolve, reopening the tear duct. Preformed plugs are difficult to insert and, because they cannot conform precisely to the natural shape of the channel, they are easily dislodged and/or deform the channel. The formation of a plug by polymerization in situ is a very difficult procedure. The composition must be delivered to exactly the right place, and, once polymerized is difficult or impossible to remove. Cyanoacrylate monomers are toxic and polymerize so rapidly that the catheter used to supply them to the channel is liable to be cemented in place before it can be withdrawn. Other procedures which have been used to prevent flow through channels in mammals include vasectomy (i.e. severing the vas deferens) to induce male sterility, and ligature of the fallopian tubes to induce female sterility. These procedures have the disadvantage that they are very difficult to reverse. It has also been proposed to insert closed mechanical valves in the vas deferens, but such valves are difficult to manufacture, difficult to insert without damage to the patient, and difficult to reopen.

We have discovered that many of the above disadvantages can be overcome through the use of a thermoplastic polymeric composition having a melting point such that (a) the composition can be delivered to the channel at an elevated temperature at which the composition is fluid but which can be tolerated by the channel, and (b) the composition solidifies as it cools to the normal temperature of the channel. Such a composition is referred to herein as an occluding composition. Preferably the composition does not undergo any change during the method except a change of state from fluid to solid.

In a first aspect, this invention provides a thermoplastic composition which is non-immunogenic and biocompatible; which is suitable for use in a method of occluding a channel in a living mammal; which is solid at the temperature of the channel; which becomes fluid when heated to a temperature at which it can be injected into the channel without damaging the cells of the mammal; and which comprises (i) a crystalline thermoplastic polymer having a crystalline melting point of 30 to 50°C, preferably 34 to 45°C, and (ii) a biologically inert additive which is opaque to X-rays, for example gold powder, which is preferably present in amount at least 25% by weight of the composition.

The composition can also contain, distributed in the polymer, at least one additive which is
(a) a particulate filler which has a specific heat of at least 0.084 kJ kg⁻¹°C⁻¹ (0.02 cal/g/°C), preferably at least 0.126 kJ kg⁻¹°C⁻¹ (0.03 cal/g/°C); or
(b) a biologically active substance, in particular a blood-coagulant, antibiotic, spermicide or growth-promoting hormone.

In a second aspect, this invention provides an injecting device which is suitable for use in a method of occluding a channel in a living mammal, said device comprising
(a) a reservoir which contains a solid, thermoplastic, non-immunogenic and biocompatible composition (14) comprising a crystalline thermoplastic polymer having a melting point of 30 to 50°C;
(b) an injection node (102) which communicates with the reservoir;
(c) pressure means (121) for exerting pressure on the occluding composition (14) so that if the composition is molten, it can be extruded through the injection nozzle (102) by operating the pressure means; and
(d) an electrical heater (12) which, when connected to a suitable power source (131), will heat the occluding composition so that it is molten.

In a third aspect, this invention relates to the use of a crystalline thermoplastic polymer having a melting point of 34 to 45°C in the formulation of a thermoplastic, non-immunogenic, biocompatible composition for occluding a channel in a living human being, the channel being a tear duct, a fallopian tube, a vas deferens, an artery, a blood vessel, or a cavity in a bone.

### Definitions, Units, Measurements and Abbreviations

The term "channel" is used herein to denote any natural or artificial passageway or cavity within a mammal, including but not limited to passageways for moving a fluid, e.g. tears, sperm, ova or blood, from one part of a mammal to another. Such channels are sometimes referred to as tubes, ducts, arteries, foramina, cavities, canals and vessels. They include fallopian tubes, naso-lacrimal ducts (also known as tear ducts or canalicular ducts), blood vessels, vas deferens, and cavities within bones, for example cranial taps and cavities for metal pins, e.g. in the lumen of the femur for hip replacement. They may include flow controls such as sphincters and capillary beds, which may be controlled voluntarily by the mammal, e.g. by muscle contractions. The mammal may be a human being or an animal, e.g. a horse, a dog or a cat.

The term "occluding" is used herein to mean completely or partially blocking, filling or enclosing. In most cases, the objective will be to block the channel completely. However, in other cases, the objective may be to provide a channel with a hollow lining which restricts but does not prevent fluid flow or which prevents the channel from collapsing, thus ensuring that fluid flow is possible. Other possible uses will be apparent to those skilled in the art having regard to the disclosure in this specification and their own knowledge.

In this specification, unless otherwise stated, parts, amounts and percentages are by weight. Temperatures are in °C. Molecular weights are in Daltons and are determined by gel permeation chromatography (GPC) in THF (tetrahydrofuran) against a polystyrene standard, the Mₙ values being number average molecular weights and the M_{w} values being weight average molecular weights. Elongation and Young's Modulus values are measured at 25°C using a tensile test instrument, for example an Instron tensile tester, at a crosshead speed of 0.5 inch/minute (1.27 cm/minute). Viscosity values are measured at 25°C and a solids content of 30% (we used a Brookfield Viscometer Model LVT) and expressed in centipoise. First order transition points (often referred to as melting points), glass transition points, and heats of fusion are determined by a differential scanning calorimeter (DSC), using the second heat cycle and a heating rate of 10°C/minute. For the polymers used in the occluding compositions, the beginning of the DSC curve (i.e. the onset of melting) is referred to as Tₒ and the peak of the DSC curve (i.e. the first order transition point) is referred to as T_{q}. For the macromers used as hard block precursors in the preparation of thermoplastic elastomers (TPE's) used in the occluding compositions, the peak of the DSC curve is referred to as Tₘ. The recrystallization time (XL time) is measured by one of two different methods. The XL time at 25°C is measured by a procedure in which (a) a steel plate having on its top surface a layer 10 microns thick of the composition is heated to 60°C, (b) the plate is placed on a cooling surface maintained at 25°C, and (c) a glass rod, 0.2 cm in diameter, is dipped into the occluding composition at frequent intervals; the recrystallization time is the time elapsed between placing the plate on the cooling surface and the time when it is no longer possible to draw a fine string of the composition from the plate with the glass rod. The XL time at 35°C is measured by a procedure in which the composition is placed in a DSC, heated to 50°C, cooled to 35°C over a period of about 4.5 secs, and then maintained at 35°C. The recrystallization time at 35°C is the time which elapses between the composition being cooled to 35°C and the time at which the maximum exotherm appears on the DSC curve.

### Occluding Compositions

A channel in a living mammal has a range of temperatures at which it normally exists, and the channel itself, or other parts of the mammal adjacent the channel, may be damaged if the channel is heated to a temperature substantially above that range. This imposes two essential requirements on the occluding compositions. First, the composition must be solid throughout the range of natural body temperatures of the channel, so that the molten composition will solidify in the channel and will not be accidentally removed by melting after it has solidified. Second, the composition must have a viscosity, not far above its melting point, which permits the molten composition to be placed in the channel at a temperature low enough to ensure that the composition does not substantially damage the mammal.

Human body temperatures are normally in the range of 36°C to 38°C, but can be as high as 42°, for example during vigorous exercise or during illness. Most human body cells can tolerate a temperature of up to about 45° for a few minutes and of up to about 50° for several seconds, and human bones can tolerate the temperatures created while polymerizing methyl methacrylate within a bone cavity, which may be about 60°C or more, for a brief period. It is preferred, therefore, that an occluding composition for use in a human should have a melting point which is at least 39°C, particularly at least 40°C, especially at least 42°C, and not more than 45°C, particularly not more than 44°C, especially not more than 43°C. It is also preferred that the composition has a viscosity between its melting point and 45°C such that the molten composition can be placed in the channel at a temperature not greater than 45°C, so that no damage is done to the mucus membrane surface of the channel to be occluded, or to other parts of the body.

Other mammals may have different natural body temperatures and different temperatures which will cause damage, and for such mammals the properties of the occluding composition should be selected accordingly. Preferably the occluding composition has a melting point which is at least 2°C above, particularly at least 3°C above, the maximum normal temperature of the channel.

The viscosity of the composition, and the rate at which it solidifies, during the process, are also important factors. A composition which has low viscosity and solidifies slowly is easy to deliver and initially conforms well to the channel, but tends to run out of (or be washed out of) the channel before it forms a plug. A composition which has high viscosity and solidifies rapidly is difficult to deliver and tends not to conform well to the channel, but is unlikely to run out of (or be washed out of) the channel. The appropriate compromise will depend mainly on the size of the channel to be occluded and the danger that the composition will run out of (or be washed out of) the channel as it solidifies. The delivery device preferably includes a heater which maintains the composition molten, but if there is no such heater, it is important to take account of the time for which the molten composition will remain in the delivery device. It is in any case important to take account of the extent to which the composition will cool (e.g. in the injection nozzle), before it reaches the channel to be occluded.

The occluding compositions used in this invention comprise a polymeric component and may also contain other ingredients. As discussed below, the other ingredients, if present, can have a significant effect on the way in which the composition flows and solidifies. However, the dominant influence is the nature of the polymeric component. The polymeric component preferably comprises a single polymer, and will chiefly be described herein with reference to a single polymer. However, the description is also applicable to a blend of two or more polymers, the blend having the same or similar properties for the purposes of the present invention. The ingredients of the occluding compositions are preferably biocompatible and non-immunogenic in order to avoid irritation or other adverse reaction of the mammal being treated.

The polymer in the occluding composition preferably has one or more of the following characteristics:
- T_{q}: 30-50°C, particularly 34-45°C, especially 38-42°C.
- T_{q}-Tₒ: Less than 10°C
- Viscosity at 50°C: Less than 1000 kgm⁻¹s⁻¹ (1,000,000 centipose) particularly 50 to 1,000 kgm⁻¹s⁻¹ (50,000 to 1,000,000, centipoise)
- Mw: 100,000 to 200,000, particularly 130,000 to 160,000

The recrystallization time is generally 1 to 10, e.g. 2 to 5, minutes at 25°C. The preferred recrystallization time depends upon the channel to be occluded. For occluding canalicular ducts, or the like, a recrystallization time at 35°C of 0.8 to 3 minutes, e.g. 1 to 1.5 minutes, is generally preferred.

The composition is preferably at a temperature from 2°C to 6°C above its first order transition point (T_{q}) when it is placed in the channel.

Preferred polymers for use in this invention are side chain crystallizable polymers (often referred to as SCC polymers). Such polymers can be broadly defined as polymers comprising repeating units of the formula 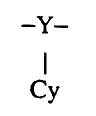 where Y is a trivalent organic radical and Cy comprises a crystalline moiety. SCC polymers include homopolymers, random copolymers containing two or more different units of the above formula, random copolymers containing one or more different units of the above formula and one or more repeating units of a different type, and block copolymers (including graft copolymers) in which at least one of the blocks is an SCC polymeric block. Particularly preferred for use in this invention are thermoplastic elastomers (often referred to as TPE's) in which at least the hard blocks are SCC polymeric blocks, especially such TPE's in which repeating units as defined above provide at least part of the backbone of polymeric blocks which extend from the main chain of the polymer.

Many SCC polymers are known. They include polymers of one or more monomers such as substituted and unsubstituted acrylates, fluoroacrylates, vinyl esters, acrylamides, maleimides, a-olefins, p-alkyl styrenes, alkylvinyl ethers, alkylethylene oxides, alkyl phosphazenes and amino acids; polyisocyanates; polyurethanes; polysilanes; polysiloxanes; and polyethers; all of such polymers containing long chain crystallizable groups. Suitable SCC's are described, for example, in the articles whose first page is given by the following references: J. Poly. Sci. 60, 19(1962), J. Poly. Sci, (Polymer Chemistry) 7, 3053 (1969), 9, 1835, 3349, 3351, 3367, 10, 1657, 3347, 18, 2197, 19, 1871, J. Poly. Sci, Poly-Physics Ed 18 2197 (1980), J. Poly. Sci, Macromol. Rev, 8, 117 (1974), Macromolecules 12, 94 (1979), 13, 12, 15, 18, 2141, 19, 611, JACS 75, 3326 (1953), 76; 6280, Polymer J 17, 991 (1985); and Poly. Sci USSR 21, 241 (1979).

In the repeating units of formula 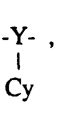 , the crystallizable moiety in the Cy radical may be connected to the polymer backbone directly or through a divalent organic or inorganic radical, e.g. an ester, carbonyl, amide, hydrocarbon (for example phenylene), amino, or ether link, or through an ionic salt linkage (for example a carboxyalkyl ammonium, sulfonium or phosphonium ion pair). The crystallizable moiety in the radical Cy may be aliphatic or aromatic, for example alkyl of at least 10 carbons, preferably 14-22 carbons, fluoralkyl of at least 6 carbons or p-alkyl styrene wherein the alkyl contains 6 to 24 carbons. The term Cₙ is used herein to denote a linear compound or group containing n carbon atoms and the term CnA is used to denote a linear alkyl acrylate wherein the alkyl group constains n carbon atoms; for example a C₂₂ alkyl acrylate is an alkyl acrylate in which the alkyl group is a linear alkyl group containing 22 carbon atoms, i.e. docosanyl (also known as behenyl) acrylate. The polymer may contain two or more different repeating units of this general formula. The polymer may also contain other repeating units, but the amount of such other units is generally at most 80%, preferably at most 50%, particularly at most 35%. The heat of fusion of the SCC polymer is generally 5 to 50 J/g, preferably at least 10 J/g, for example 10 to 35 J/g.

In the SCC polymers used in this invention, it is preferred that the total number of carbon atoms in the side chains is at least 4 times, e.g. at least 5 times, the total number of carbon atoms in the backbone. Preferred side chains comprise linear polymethylene moieties containing 14 to 22 carbon atoms. Polymers containing such side chains can be prepared by polymerising one or more corresponding linear aliphatic acrylates, methacrylates, acrylamides or methacrylamides, optionally with one or more other comonomers preferably selected from other alkyl, hydroxyalkyl and alkoxyalkyl acrylates, methacrylates (e.g. glycidal methacrylate), acrylamides and methacrylamides: acrylic and methacrylic acids; acrylamide; methacrylamide; maleic anhydride; and comonomers containing amine groups. Such other monomers are generally present in total amount less than 50%, particularly less than 35%, especially less than 25%, e.g. 0 to 15%. When acrylic acid is used, the amount thereof is preferably less than 10%. They may be added to modily the melting point, or adhesiveness, or other physical properties of the polymer. The melting point of a polymer containing such polymethylene side chains is determined chiefly by the number of carbon atoms in the crystallizable side chains, and this should be remembered in selecting polymers for use in this invention. For example, homopolymers of n-alkyl acrylates in which the alkyl groups contain 14, 16, 18, 20, 22, 30, 40 and 50 carbons respectively typically have melting points of 20, 36, 49, 60, 71, 76, 96 and 102°C, while the homopolymers of the corresponding n-alkyl methacrylates typically have melting points of 10, 26, 39, 50, 62, 68, 91 and 95°C. Copolymers of such monomers generally have intermediate melting points. Copolymers with other monomers, e.g. acrylic acid or butyl acrylate, typically have somewhat lower melting points.

In one class of SCC polymers, the repeating units of formula -Y(Cy)- have the formula 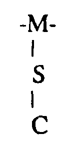 wherein
M is -(CH₂)ₘ-CH-, where m is 0, 1 or 2;
S is -O-, -CH₂- 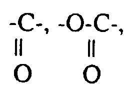 or -NR-where
R is hydrogen or alkyl containing 1 to 16 carbons; and
C is -(CH₂)ₙCH₃ or -(CF₂)ₙCF₂H, where n is 6 to 21 inclusive.

Specific examples of SCC homopolymers which could be used in occluding compositions include polymers of the following monomers, the melting points of those polymers being given in parentheses: dodecylvinyl ether (33°), 1,1-dihydroperfluorooctylacrylate (35°), caprylaldehyde (35°), 1-decene (40°), trans-1,2-dichloro-dodecamethylene (40°), vinyl palmitate (41°), hexadecyl-acrylate (43°), 1-dodecene (45°) and N-hexadecyl-acrylamide (45°).

As briefly noted above, particularly preferred SCC polymers for use in this invention are TPE's containing SCC polymeric blocks. Such TPE's (many of which are novel) are described in detail in copending, commonly assigned US Application Serial Nos. 07/773, 047, 07/957,270 and 08/048,280, and the corresponding International (PCT) application No. PCT/US92/08508 filed Cctober 6, 1992

SCC TPE's are preferred to simple SCC homopolymers or random copolymers because they have better physical properties (in particular are less brittle) at mammalian body temperatures, and because the rate at which they crystallize can be adjusted by changing the proportion of SCC polymer hard blocks in the TPE.

In a preferred class of SCC TPE's, the soft blocks are derived from an amorphous polymer and the hard blocks are derived from an SCC polymer. The TPE will generally contain 10 to 80%, preferably 15 to 60%, particularly 30 to 50%, of the hard block. The SCC hard blocks can be derived from SCC polymers as described above, particularly those prepared by the polymerization of long chain acrylates, or the like, optionally with one or more other monomers. The soft blocks are preferably polyacrylates, this term being used to include polymers of at least one alkyl acrylate, methacrylate, acrylamide or methacrylamide, optionally with other copolymerizable monomers such as acrylic acid, methacrylic acid, acrylamide, methacrylamide, acrylonitrile, acrolein, vinyl esters and styrene. Thus we have obtained excellent results using an SCC TPE in which the soft block is an amorphous block derived from butyl acrylate and the hard block is derived from a random copolymer of docosanyl acrylate and stearyl acrylate (also known as octadecyl acrylate).

In another class of SCC TPE's, the hard blocks are derived from a first SCC polymer and the soft blocks are derived from a second, lower melting, SCC polymer. Such a TPE will be a rigid solid below the melting point of the second SCC polymer block, an elastomer between the melting points of the first and second SCC polymers, and a viscous liquid above the melting point of the rust SCC polymer.

The melting point of the SCC TPE's is determined chiefly by the melting point of the SCC hard block, but as noted above, the recrystallization time depends chiefly on the proportion of the SCC hard block. For example TPE's which have poly(butyl acrylate) soft blocks and SCC hard blocks derived from octadecyl and hexadecyl acrylates have recrystallization times at 25°C of about 16, about 97, and about 154, seconds at SCC hard block concentrations of about 40%, about 30%, and about 20%, respectively.

It is also possible to make an SCC TPE which has two different SCC hard blocks. Such an SCC TPE will show bimodal melting behavior.

The polymer in the occluding composition can also be a conventional crystalline polymer, i.e. one whose crystallinity results from main chain crystallization. Most main chain crystallizable polymers (MCCP's) have melting points which are too high for use in this invention, e.g. 75° to 320°C. However, there are some MCCP's which have melting points in the preferred range of 30° to 50°C. Preferred MCCP's for use in this invention include water-insoluble polyalkylene oxides, lower alkyl polyesters, and polytetrahydrofuran (T_{q} 37°C). Other suitable MCCP's are low molecular weight fractions (often referred to as oligomers) of polymers which normally have higher molecular weights and higher melting points. For example oligomers of the formula H(CH₂)ₙH have melting points of about 32-34°C, 36-38°C, 40-42°C and 43-45°C, when n is 19, 20, 21 and 22 respectively, while polyethylene in which n is more than 1000 has a melting point of 143-145°C. When the polymer is a polyolefin which can exist in different tactic forms, it is important to use a polymer which is in only one of those forms (atactic, syndrotactic or isotactic), so that the melting point of the polymer is sufficiently sharp.

Specific examples of MCCP's with melting points in 30-50°C range include homopolymers of the following monomers, the melting points of those polymers being given in parentheses. Copolymers of two or more such monomers will have similar melting points.

2, 2, 3, 3, 4, 4-hexafluoro-(diamine)-pentamethylene adipate (34°), tetramethylene succinate (34°), N,N'-diethyl-4,4'-methyenediphenylene sebacamide (34°), trimethylene malonate (34°),difluoro-methylene sulfide (35°), N,N'-diisopropyl 2, 2, 3, 3, 4, 4-hexafluoro-(diamine)-pentamethylene adipamide (35°), trimethylene oxide (36°), cis-2 methyl-1,3-butadiene (36°), 4,methyl-(R+)-7, hydroxyenanthic acid (36°), trimethylene pimelate (37°), hexamethylenedithiotetramethylene disulfide (38°), hexamethyleneoxymethylene oxide (38°), trimethylene glutarate (39°), tetramethylene disulfide (39°), methyleneoxypentamethylene oxide (39°), diethyl-dimethyl-(Si)-O-phenylene disiloxanylenedipropionamide (40°), O-phenylene disiloxanylenedipropionamide (40°), N,N'-diethyl-4,4'-methyelnediphenylene azelaamide (41°), trimethylene suberate (41°), cis-1,4-cyclohexylenedimethylene azelaate (41°), pentamethylene azelaate (41°), trans-1,4-cyclohexylenedimethylene pimelate (42°), tetrafluoro-ethylene oxide (42°), isobutene (44°), isotactic cis-1,3-pentadiene (44°), pentamethylene disulfide (44°), oxydiethylene sebacate (44°), cyclopropylidenedimethylene oxide (45°), ethylene p-(carboxyphenoxy)-caproate (45°), N,N'-dibutyl-3,3'-dimethyl-(diamine)-4,4'-methyenediphenylene adipamide (45°), N,N'-diisoamyl-3,3'-dimethyl-(diamine)-4,4'-methylene diphenylene adipamide (45°), decamethylene disulfide (45°), trimethylene adipate (45°), and 2,2-dimethyl-(diol)-trimethylene adipate (45°).

In some cases, it is desirable for the occluding composition to have good adhesion under moist conditions, e.g. to moist skin. In these cases, the polymer can be made more polar by incorporation of polyoxyalkylene units, e.g. polyoxyethylene, polyoxypropylene or polyoxybutylene units.

In some cases, it is desirable for the occluding composition to have a good moisture vapor transmission rate (MVTR). In these cases, the polymer preferably contains units derived from one or more hydrophilic monomers, e.g. acrylic acid; acrylamide; hydroxyalkyl acrylates and methacrylates such as hydroxyethyl acrylate and methacrylate and hydroxybutyl acrylate; alkoxy acrylates and methacrylates such as ethoxyethyl acrylate, ethoxyethoxy ethylacrylate, ethyltriglycol methacrylate, and 3-methoxybutyl acrylate; derivatives of polyethylene glycol with molecular weights ranging from 50 to 5,000. These units may be inc orporated either into the backbone or as pendant groups.

Moisture vapor transmission rates and/or absorptive properties of the occluding compositions may also be modified by the incorporation of soluble or insoluble hydrophilic materials, for example carboxymethyl cellulose, guar gum, carragenan, and cellulose.

As briefly noted above, the occluding compositions used in this invention can include other ingredients in addition to the polymer. Such other ingredients include:
(a) Solid particulate fillers; such fillers can be used for example to modify the properties of the composition, in particular the opacity of the composition to x-rays, the physical properties of the solid composition (e.g. tensile strength, impact strength, and elongation), or the physical properties of the composition in the melt or in the transition between solid and melt; thus the filler can act as a heat sink, as a reinforcing filler, as a viscosity modifier, or as a site for initiating crystallization. Such fillers are preferably biologically inert, and include for example radio-opaque pigments in general, gold powder, silver powder, and fumed silica. The filler can be dispersed in the polymer by dissolving the polymer in a suitable solvent, e.g. pentane or toluene, at a temperature above the melting point of the polymer; mixing the filler with the solution; and maintaining the dispersion at an elevated temperature, with constant agitation, until all the solvent has evaporated.
(b) Biologically active substances which are distributed in (including chemically attached to) the polymer, and which have a desire effect on the mammal after the occluding composition has solidified. The active substance may for example be released from the plug of occluding composition on a regular or irregular schedule by natural functions of the body, e.g. by being leached out by natural body fluids, or by some artificial means, for example by fluids introduced into the body for this purpose. Such substances include for example blood coagulating compounds, antibiotics, spermicides, and growth-promoting hormones.

Because the rate at which the occluding composition cools is important, the presence of a filler which acts as a heat sink can be very significant. For example the occluding composition can contain 25 to 95%, preferably 40 to 85%, particularly 50 to 80%, by weight, based on the weight of the composition, of a particulate filler having a specific heat of at least 0.084 kJkg⁻¹°C⁻¹ (0.02 cal/g/°C), preferably at least 0.126 kJkg⁻¹°C⁻¹ (0.03 cal/g/°C). A preferred filler is gold powder, preferably having a diameter of 0.5 to 10 µm, e.g. 1 to 3 µm, and preferably in amount 60-80%.

Because the viscosity of the molten composition is important, the presence of a filler which modifies its viscosity can be very significant. For example, fumed silica can be used to increase the viscosity of the molten composition.

### Sterilization

In order to make sterile compositions, known sterilization procedures can be employed. It should be noted, however, that the use of radiation can result in an increase or a decrease in the molecular weight of the polymer, depending on the polymer and the conditions, in particular the temperature, of the radiation. This fact can be put to use in modifying the properties of the composition in a desired way. In general, however, it is preferred to use heat sterilization or another method which does not substantially change the composition.

### Injecting Devices

When the channel to be occluded is one which, under the conditions of treatment, is easily accessible (either because it is at or near the surface of the body, or has been exposed in the course of an invasive operation, it is preferred to make use of an injecting device comprising (a) a reservoir in which an occluding composition can be maintained in a molten state (sometimes referred to herein as the "preplug") (b) an injection nozzle which communicates with the reservoir and (c) means for exerting pressure on a molten occluding composition in the reservoir so that the composition is extruded through the injection nozzle. Preferably the capacity of the reservoir is equal to the amount of the composition needed for a single use, e.g. 0.0005 to 0.0015 mL. Preferably, the reservoir is filled some time before the device is to be used by placing molten occluding composition in the reservoir, and allowing it to solidify in the reservoir. The device and the composition are generally sterilized together after the composition has been placed in the device. However, it is also possible to sterilize the device and the composition separately, and then to place the composition in the device under sterile (called "clean fill") conditions. The filled device is of course maintained under sterile conditions until it is used. At the time of use, the composition is re-melted so that it can be extruded through the nozzle. This remelting can be effected by heating the whole device, e.g. in a water bath, oil bath or oven, or by operation of an electrical heater which forms part of the device. The device can also include an electrical heater which maintains an already molten occluding composition in the molten state, for example a heater which heats the composition as it is being extruded through the nozzle. When the device includes a heater, the heater can be powered by a battery which forms part of the device, or by an external power source. The device can include a means for indicating when the occluding composition is at a suitable temperature, for example a pin which is embedded in the solid composition, e.g. through the injection nozzle, and which can be easily withdrawn when the composition has melted, but not before. The device can be one which is designed for use a single time or multiple times.

When the channel to be occluded is not easily accessible, but can be reached by a catheter or the like, the invention preferably makes use of a catheter which is fitted with an electrical heater at its tip. The catheter is pushed through the vessels of the circulatory system until the tip of the catheter has reached the channel to be occluded. The occluding composition, in the form of a fine filament, is placed in the catheter before the catheter is pushed into position, or is pushed through the catheter after the catheter has been pushed into position. The heater at the tip of the catheter is used to melt the occluding composition as it is pushed out of the catheter into the channel.

The preferred outer diameter of the injection nozzle or catheter will depend on the channel to be occluded, and the preferred inner diameter will depend on the rate at which the composition is to be delivered. For example, to block a canaliculus, a 24 gauge needle (internal diameter 0.037 cm, external diameter 0.057 cm) is generally satisfactory because it fits easily within the opening of the punctum which leads to the canaliculus; to block fallopian tubes, a 2 mm catheter (internal diameter 0.18 cm, external diameter 0.21 cm) is generally satisfactory; to fill a bone cavity, a 0.64 cm cannula (internal diameter about 0.5 cm, external diameter 0.64 cm) is generally satisfactory.

The tip of the injection or nozzle can be shaped for ease of insertion and use. For example, when blocking a canaliculus, a tip having an angle of about 9°, e.g. 6 to 12°, has been found to be particularly satisfactory.

Injection devices of many kinds are well known in the medical profession, including plastic tubes, catheters, cannula (including tapered cannula), syringes and hypodermic syringes, and those skilled in the art will have no difficulty, after consideration of the disclosure in this specification, in making injection devices suitable for use in this invention.

### Methods of Use

The compositions used in this invention are particularly useful for occluding tear ducts to relieve dry eye syndrome. The occluding composition can be injected through the upper punctum to occlude the upper canalicular channel, or through the lower punctum to occlude the lower canalicular channel, or both. It is also useful for occluding fallopian tubes to prevent pregnancy; occluding vas deferens to induce sterility; occluding blood vessels to close off blood supply, e.g. to block blood flow to a tumor area or to plug up a hemorrhage in the brain, liver, kidney, or spleen or to correct an abnormality such as arteriovenous anastomosis; occluding a man-made channel, e.g. a temporary cranial tap to release blood; and occluding bone cavities for pins, particularly for steel pins or spikes in the natural lumen of a finger or limb bone, e.g. in a hip joint replacement. In the last method, preferably the molten occluding composition is placed in the bone cavity, and while the composition is still hot, the pin or spike, preheated to an appropriate temperature, is pushed into the cavity, displacing the composition so that it flows around the pin or spike and conforms to it and the walls of the channel. Those skilled in the art will readily recognize, after reading this specification, other situations in which the present invention can be usefully employed.

The amount of the occluding composition which is placed in the channel will of course vary substantially. For example, in the treatment of dry eye, the solidified plug will usually be about 8 to 12 mm long and about 1-2 mm in diameter. The weight of such a plug, when using a composition containing about 75% of gold powder, is about 40 to 60 g. The time taken to inject the occluding composition should generally be short. For example, an injection time of no more than about 3 seconds is preferred.

The molten occluding composition cools and solidifies in the channel, thus forming a plug which at least partially blocks the channel. Preferably the composition completely fills and closely conforms to the channel before it cools, thus forming a plug which completely blocks the channel, but does not dilate it. Preferably the plug is composed of a material which remains solid under normal body conditions and which thus remains in place until positive steps are taken to remove it.

A particular advantage of the occluding compositions used in this invention is that if it is desired to remove the plug, and thus to reopen the channel, this can be done by simple and reliable methods. In some cases, the solid plug can be extracted from the channel with a suitable gripping device, or pushed through the channel into another part of the body where it will do no harm. In most cases, however, the plug will be heated and/or contacted by a liquid composition which at least partially dissolves the composition, so that the composition becomes flowable. A preferred liquid composition for this purpose is a lipophilic composition comprising an oil, preferably a natural oil, or a fatty acid ester, which dissolves into the composition and reduces its melting point. The composition can then be removed from the body, e.g. by gentile suction or gravitational flow, or with the aid of a suitable gripping device, e.g. a cold fine wire which is pushed into the plug so that a portion of the plug cools and is thus secured to the wire, and is then withdrawn. Alternatively the composition can flow into another part of the body where it will do no harm. The removal of the composition from the channel can be assisted by the flow of natural body fluids, and/or by the introduction of a fluid, e.g. a saline solution or air, for this purpose.

As previously noted, a preferred use of the compositions used in this invention is to treat dry eye syndrome. Dry eye syndrome is sometimes caused by a failure of the lacrimal glands to produce tears, or by blockage of the tear ducts from the lacrimal glands. When this is the cause, the compositions cannot be used to correct it. In most cases, however, tear production is adequate (even if less than normal) and dry eye syndrome is caused by excessive drainage of the tears through the upper and lower punctal openings which lead to the canalicular channels. The compositions used in this invention provide an excellent treatment for dry eye caused by excessive drainage, since it can be used to occlude one or more of the canalicular channels. Furthermore, the plugs can be easily removed if it should be found that the cause of the dry eye is not excessive drainage, but inadequate tear production. The temperature of the occluding composition, as it enters the punctum preferably does not exceed 42°C.

When the compositions used in this invention are used for occluding fallopian tubes or vas diferens, it is most important that the channel should be completely blocked. Preferably, therefore, the channel is filled over most or all of its length. The normal temperature of the vas deferens is slightly below 37°C, and this should be remembered when using the invention to occlude the vas deferens or to remove an existing plug in the vas deferens.

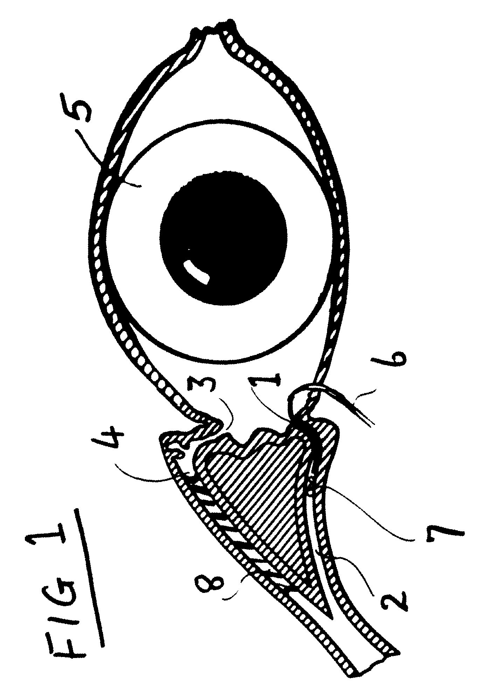

The use of the compositions used in this invention is illustrated in the accompanying drawings. Figure 1 is a diagrammatic cross-section of the lacrimal duct system of a human eye 5 in which the lower canalicular channel is being occluded. The eye includes lower punctum 1 which leads to lower canalicular channel 2, and upper punctum 3 which leads to upper canalicular channel 4. Tears drain from the eye through the canalicular channels, and if the rate of drainage exceeds the rate of tear production, the eye suffers from dry eye syndrome. A flexible nozzle 6 forms part of an injection device (not otherwise shown). Molten occluding composition 7 is being injected into the lower canaliculus. The upper canaliculus is already blocked by a plug 8 of solidified occluding composition previously injected through upper punctum 3.

Figure 2 shows an injection device suitable for injecting an occluding composition into a canalicular channel in the treatment of dry eye. The device comprises a stainless steel tube 101 which has been drawn out to form an injection nozzle 102 having an angled tip. The tube could be formed for example from a 6.3 cm length of 19 gauge XTW stainless steel (inner diameter about 0.08 cm) having one end drawn down to an outer diameter of about 0.06 cm and an inner diameter of about 0.025 cm. The tube is surrounded by a first layer 111 of insulation, around which is wrapped an electrical resistance wire heater 12. The heater 12 is encased in a second layer 112 of insulation. The heater 12 is connected via leads 13 to a battery 131 within a case 132. The tube 101 forms a reservoir for occluding composition 14. A plunger 121 closes the end of the tube 101 and can be operated by means of arm 122. A pin 140 closes the nozzle 102. In operation, the heater is switched on to melt the composition 14, and, when the device is to be used, the pin 140 is withdrawn from the nozzle and the composition is extruded through the nozzle by operating the plunger 121.

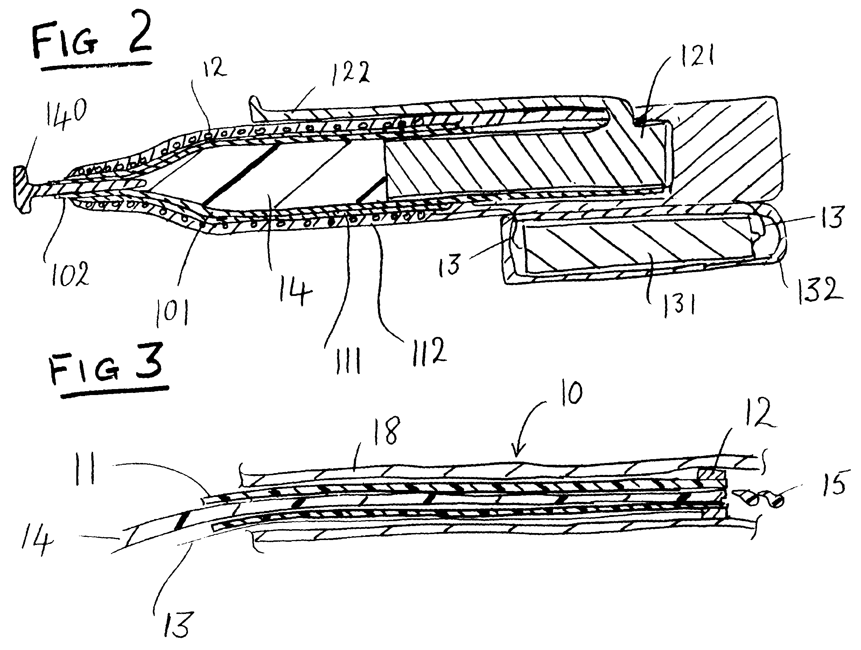

Referring now to Figure 3, a catheter 10 comprises a guided catheter tube 11, a small electrical heater 12 which fits around the tip of the catheter, and lead wires 13 which carry power to the heater 12. The catheter 10 has been pushed through a vein 18 until its tip reaches the site to be occluded. A solid filament 14 of occluding composition has been pushed through the tube 11, after the catheter has been put into position. The filament is fed through the heated tip of the catheter at a rate such that it melts to form a liquid 15 as it emerges from the tip of the catheter.

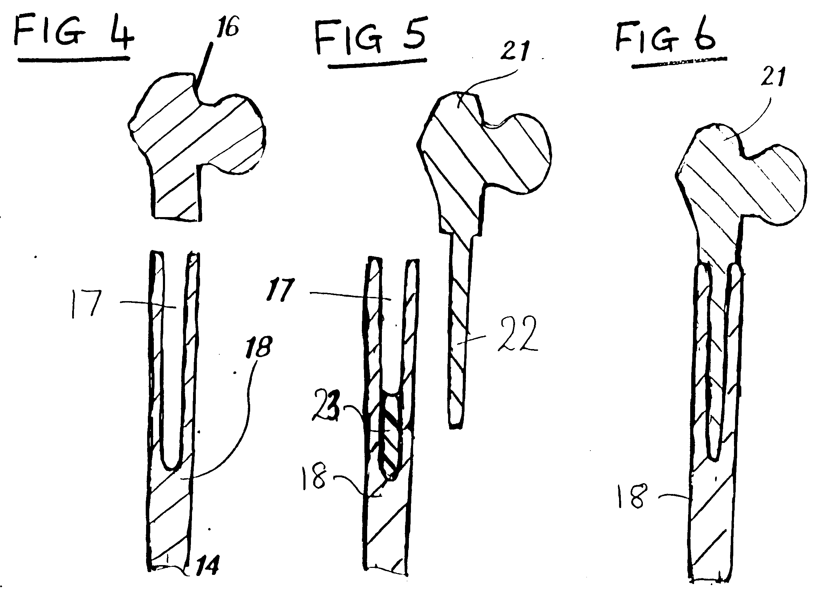

Referring now to Figures 4, 5 and 6, these show successive stages in the replacement of a defective femoral ball end 16 by an artificial ball end 21. First, as shown in Figure 4, the defective ball end is removed. Next the channel 17 within the femor 18 is enlarged so that the pin 22 of the artificial ball end 21 can fit into it. As shown in Figure 5, a quantity of molten occluding composition 23, e.g. at a temperature of about 50°C, is placed in the enlarged channel. Immediately thereafter the pin 22, preheated to a suitable temperature, e.g. about 50°C, is pushed into the channel, forcing the molten occluding composition to flow around it and along the channel walls, completely filling the channel, as shown in Figure 6, The ball end is held in precisely the correct position until the composition has solidified, thus locking the ball end in place.

The invention is further illustrated by the following Examples.

### Examples 1-9

In Examples 1-9, the ingredients and amounts thereof shown in Table 1 were used to provide compositions of the invention.

In Example 1, the polymer and filler were added to toluene (100 parts), the mixture heated to about 75°C, and the solvent removed under vacuum while the mixture was maintained at about 70°C. Similar procedures were used in Examples 1, 2, 6 and 9 to disperse the filler in the polymer.

The following tests were carried out.

The composition of Example 1 was heated to 50°C; a glass rod was placed in the composition while it was at 50°C; the viscous composition was cooled to about 35°C and at that temperature, the rod was locked in the composition, which was now solid; the composition was reheated to 45°C, when the rod could be extracted from the viscous composition, thickly coated with composition; the coated rod was cooled to room temperature. The composition was opaque to radio waves, and appeared suitable for anchoring a bone pine in a bone cavity. However, its viscosity was somewhat greater and its rate of crystallization somewhat lower than the ideal; a lower molecular weight material would be expected to give better results.

Each of the compositions of Examples 2-6 was heated above its melting point and could then be extruded through a 16 gauge hypodermic needle (inner diameter about 0.14 cm) and, on cooling, crystallized rapidly when it reached a temperature just below the melting point.

In Example 7, the composition was heated to about 50°C and placed in a syringe filled with a blunted 24 gauge needle (outer diameter about 0.06 cm, inner diameter about 0.035 cm). About 10 mg of the hot composition (which had a viscosity of about 22 centipoise at 50°C and about 21 centipoise at 45°C) was then injected into the punctum of the superior canaliculus of a tranquilized dog. The composition immediately solidified. A similar procedure was attempted with the compositions of Examples 8 and 9. In Example 8 the gold filler had a tendency to settle out, and though the fumed silica in Example 9 reduced this tendency, it also increased the viscosity, so that the composition was more difficult to extrude.

### Examples 10-17

In Examples 10-17, the ingredients and amounts thereof shown in Table 2 were used to make SCC polymers suitable for use in the invention.

In Example 10, a mixture of the listed ingredients was added at a rate of 10 ml/min to a reactor maintained at 100°C, together with t-butyl peroctoate at 0.15 ml/min. After the addition was complete, the reactor was maintained at 100°C for 2 hours and then cooled. The product was purified by recrystallization from a toluene/ethanol mixture, followed by drying under vacuum (yield 80 parts, residual C18A 374 ppm, residual C4A, less than 130 ppm, viscosity 1530 cps at 50°C, 2300 cps at 45°C).

A similar procedure was followed in Examples 11-17.

### Examples 18-25

Examples 18-25 show the preparation of functionalized SCC polymers ("macromers") which can be used to provide the hard blocks in TPE's suitable for use in the invention. The ingredients and amounts thereof shown in Table 3 were used to make SCC polymers having the properties shown in Table 3. These SCC polymers were then functionalized by reaction with isocyanatoethyl methacrylate (IEMA).

In Example 18, the SCC polymer was prepared by adding the C16A (8 g) and C18A (92 g), together with mercaptoethanol (3.6 g) as capping agent and AIBN (1 g) as initiator, to toluene (200 mL), and heating the reaction mixture under nitrogen at 60°C for 14 hours with stirring, and then at a higher temperature to destroy residual AIBN. After cooling, IEMA (8.5 g) and dibutyl tin dilaurate (1 drop) were added, and the reaction mixture stirred at room temperature for 16 hours. The functionalized SCC polymer was precipitated by the addition of ethanol, filtered and dried under reduced pressure. The ethanol extracts residual monomers and reacts with any residual IEMA.

A similar procedure was followed in Examples 19-25, with appropriate modifications, e.g. to control the molecular weight.

### Examples 26-42

In Examples 26-42, the macromers made in Examples 18-25 were used to make TPE's by reaction with butyl acrylate, using the ingredients and amounts thereof shown in Table 4.

The TPE of Example 26 was prepared by adding the macromer of Example 8 (40 g), the butyl acrylate (60 g), dodecyl mercaptan (0.4 g), and AIBN (1 g) to toluene (200 mL), and heating at 60°C under nitrogen for 14 hours with stirring. The reaction mixture was cooled and poured into ethanol to recover the TPE as a precipitate, and the precipitate was dried at elevated temperature under reduced pressure (yield 80 g, residual C18MA 562 ppm, residual C4A 624 pm, viscosity 7200 cps at 50°C, 16,000) cps at 46°C).

The TPE's of Examples 27 to 42 were prepared similarly.

### Example 43

The TPE of Example 41 (100 parts) was blended with gold powder (spherical particles, 1.5 - 3.0 micron, 250 parts) by a procedure similar to Example 1. The composition was heated to 50°C and loaded into a small hydraulic applicator whose exit port was a No. 80 drill hole (diameter about 0.03 cm). After cooling to 23°C, it was impossible to force any of the composition out of the exit port. The applicator and composition were then heated to about 50°C, and about 10 mg of the composition was injected into the punctum of the superior canaliculus of a tranquillized dog. The composition formed a plug which could be seen under X-rays and which was in the same place after 60 days, at which time it was washed out with isotonic sterile saline solution at 46°C.

### Example 44

The procedure of Example 43 was followed using the TPE of Example 33. The plug could be seen under X-rays after 3 hours but not after 48 hours, presumably because the temperature of the canaliculus was high enough to prevent the composition from solidifying.

### Example 45

The TPE of Example 42 (2.5 g) was dissolved in 10 g of pentane (Omni-Solv Px0167) using a sonic agitator and replacing any pentane which evaporates. To this solution was added 7.5 g of gold powder (1.5-3.0 micron in diameter, purity 99.9+%, available from Leico Industries Inc.). The mixture was maintained at about 50°C, with constant agitation, for about 3 hours, at which time all the pentane had evaporated. The resulting filled polymer, after it had been sterilized, was suitable for occluding the canaliculus of a human being

**TABLE 1**

| | EXAMPLE NO. | | | | | | | | |
|---|---|---|---|---|---|---|---|---|---|
| **INGREDIENTS** | 1 | 2 | 3 | 4 | 5 | 6 | 7 | 8 | 9 |
| Poly(cis-isoprene)¹ | 93 | - | - | - | - | - | - | - | - |
| Poly(tetrahydrofuran)² | - | 100 | 100 | - | - | - | - | - | - |
| Poly(trimethylene glutarate)³ | - | - | - | 100 | 100 | - | - | - | - |
| Poly(trimethylene adipate)³ | - | - | - | - | | 100 | - | - | - |
| Heneicosane⁴ | - | - | - | - | | - | 100 | 100 | 100 |
| Silver Powder | 7 | - | - | - | | 150 | - | - | - |
| Gold Powder⁵ | - | - | - | - | 250 | - | - | 300 | 300 |
| Fumed Silica⁶ | - | 10 | - | - | - | - | - | - | 10 |
| Melting Point °C | - | 43 | 37 | 40 | 40 | 45 | 43 | 37 | 40 |

| | | | | | | | | | |
|---|---|---|---|---|---|---|---|---|---|
| 1. Molecular weight about 800,000, available from Aldrich Chemical Co. | | | | | | | | | |
| 2. Available from PolySciences. | | | | | | | | | |
| 3. Available from Monomer-Polymer and Dajac Laboratories Inc. | | | | | | | | | |
| 4. CH₃ (CH₂)₁₉ CH₃, available from Aldrich Chemical Co. | | | | | | | | | |
| 5. Spherical particles of diameter 1.5-3.0 micron. | | | | | | | | | |
| 6. SiO₂, particle size about 0.007 micron. | | | | | | | | | |

**TABLE 2**

| | EXAMPLE NO. | | | | | | | |
|---|---|---|---|---|---|---|---|---|
| **INGREDIENTS** | 10 | 11 | 12 | 13 | 14 | 15 | 16 | 17 |
| Octadecyl acrylate¹ | 84 | 96 | 92 | 89 | 88 | 86 | 80 | 76 |
| Butyl Acrylate | 16 | 4 | 8 | 11 | 12 | 14 | 20 | 24 |
| Dodecyl Mercaptan | 1.86 | 1.86 | 1.86 | 1.86 | 1.86 | 1.86 | 1.86 | 1.86 |

| **PROPERTIES** | | | | | | | | |
|---|---|---|---|---|---|---|---|---|
| Mw | 19k | 17k | 19.5k | 20.5k | 22k | 31k | 18k | 18k |
| M_{w}/Mₙ | 1.6 | | | | | | | |
| DSC peak °C (Tₘ) | 38.6 | 44.5 | 44.5 | 40.2 | 40.6 | 38.4 | 36.3 | 35.0 |

| | | | | | | | | |
|---|---|---|---|---|---|---|---|---|
| 1. A mixture of long chain alkyl acrylates containing at least 90% octadecyl acrylate with the remaining alkyl radicals containing at least 14 carbon atoms. | | | | | | | | |

**TABLE 3**

| | EXAMPLE NO. | | | | | | | |
|---|---|---|---|---|---|---|---|---|
| **INGREDIENTS** | 18 | 19 | 20 | 21 | 22 | 23 | 24 | 25 |
| Hexadecyl acrylate | 8 | - | - | - | - | - | - | - |
| Octadecyl acrylate | 92 | 100 | - | 15 | 25 | - | - | - |
| Octadecyl methacrylate | - | - | 100 | 85 | 75 | 66 | 60 | 50 |
| Docosanyl methacrylate | - | - | - | - | - | 34 | 40 | 50 |

| **PROPERTIES** | | | | | | | | |
|---|---|---|---|---|---|---|---|---|
| M_{w} | 3900 | 3900 | 3900 | 3600 | 3900 | 4300 | 3400 | 4200 |
| M_{w}/Mₙ | 1.34 | 1.33 | 1.32 | 1.32 | 1.33 | 1.3 | - | 1.25 |
| DSC onset °C | 39 | 42 | 33 | 33 | 33 | 34 | 37.5 | 40.5 |
| DSC peak °C (Tₘ) | 42 | 45 | 36 | 36 | 37 | 42 | - | 44.2 |
| Heat of fusion J/g | 83 | 118 | 74 | 76 | 79 | 69 | - | 79 |

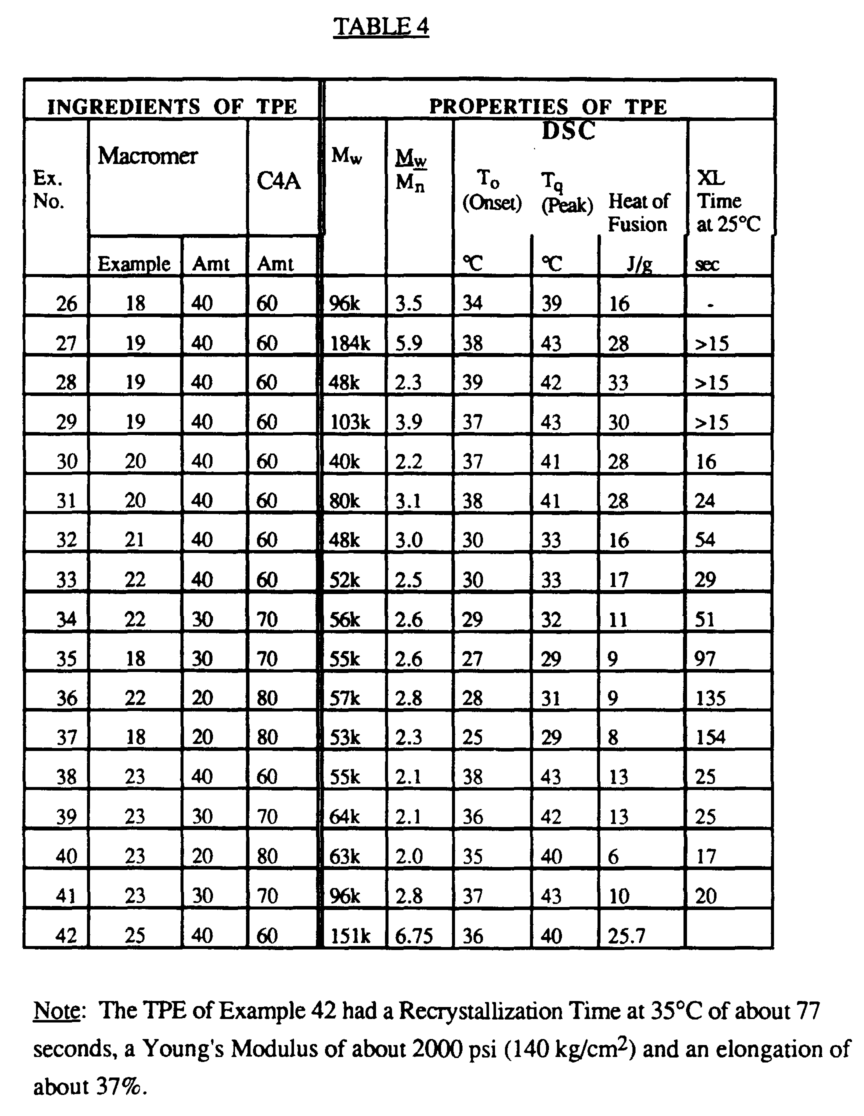

## Claims

1. A thermoplastic composition which is non-immunogenic and biocompatible; which is suitable for use in a method of occluding a channel in a living mammal; which is solid at the temperature of the channel; which becomes fluid when heated to a temperature at which it can be injected into the channel without damaging the cells of the mammal; and which comprises (i) a crystalline thermoplastic polymer having a crystalline melting point of 30 to 50°C, preferably 34 to 45°C, and (ii) a biologically inert additive which is opaque to X-rays.

2. A composition according to claim 1 wherein the additive is gold powder.

3. A composition according to claim 1 or 2 wherein the crystalline polymer comprises a side chain crystalline polymer.

4. A composition according to claim 3 wherein the crystalline polymer is a thermoplastic elastomer.

5. A composition according to claim 3 or 4 wherein the side chain crystalline polymer comprises units derived from at least one alkyl acrylate or methacrylate wherein the alkyl group contains 14-22 carbon atoms.

6. A composition according to any one of the preceding claims wherein the composition comprises at least one biologically active substance, in particular a blood-coagulant, antibiotic, spermicide, growth-promoting hormone or antibiotic.

7. A composition according to any of claims 1 to 6 suitable for use in occluding a tear duct in a human being, the composition having a melting point of at least 39°C and not more than 45°C.

8. An injecting device which is suitable for use in a method of occluding a channel in a living mammal, said device comprising
(a) a reservoir which contains a solid, thermoplastic, non-immunogenic and biocompatible composition (14) comprising a crystalline thermoplastic polymer having a melting point of 30 to 50°C;
(b) an injection nozzle (102) which communicates with the reservoir;
(c) pressure means (121) for exerting pressure on the occluding composition (14) so that if the composition is molten, it can be extruded through the injection nozzle (102) by operating the pressure means; and
(d) an electrical heater (12) which, when connected to a suitable power source (131), will heat the occluding composition so that it is molten.

9. An injecting device according to claim 8 wherein the occluding composition is a composition as claimed in any one of claims 1 to 7.

10. The use of a crystalline thermoplastic polymer having a melting point of 34 to 45°C in the formulation of a thermoplastic, non-immunogenic, biocompatible composition for occluding a channel in a living human being, the channel being a tear duct, a fallopian tube, a vas deferens, an artery, a blood vessel, or a cavity in a bone.

11. The use according to claim 10 wherein the crystalline polymer is a polymer as defined in any one of claims 3 to 5.

## Patentansprüche

1. Thermoplastische Zusammensetzung, die nicht-immunogen und biokompatibel ist, für die Verwendung in einem Verfahren des Okkludierens eines Kanals in einem lebenden Säugetier geeignet ist, bei der Temperatur des Kanals fest ist, flüssig wird, wenn sie auf eine Temperatur erhitzt wird, bei der sie in den Kanal injiziert werden kann, ohne die Zellen des Säugetiers zu beschädigen, und (i) ein kristallines thermoplastisches Polymer mit einem kristallinen Schmelzpunkt von 30 bis 50 °C, vorzugsweise 34 bis 45°C, und (ii) ein biologisch inertes Additiv umfaßt, das gegenüber Röntgenstrahlen opak ist.

2. Zusammensetzung nach Anspruch 1, bei der das Additiv Goldpulver ist.

3. Zusammensetzung nach Anspruch 1 oder 2, bei der das kristalline Polymer ein seitenkettenkristallines Polymer umfaßt.

4. Zusammensetzung nach Anspruch 3, bei der das kristalline Polymer ein thermoplastisches Elastomer ist.

5. Zusammensetzung nach Anspruch 3 oder 4, bei der das seitenkettenkristalline Polymer Einheiten umfaßt, die sich von mindestens einem Alkylacrylat oder -methacrylat ableiten, wobei die Alkylgruppe 14 bis 22 Kohlenstoffatome enthält.

6. Zusammensetzung nach einem der vorhergehenden Ansprüche, bei der die Zusammensetzung mindestens eine biologisch aktive Substanz umfaßt, insbesondere ein Blutkoagulierungsmittel, ein Antibiotikum, ein Spermizid, ein wachstumsförderndes Hormon oder Antibiotikum.

7. Zusammensetzung nach einem der Ansprüche 1 bis 6, die für die Verwendung beim Okkludieren eines Tränenkanals in einem Menschen geeignet ist, wobei die Zusammensetzung einen Schmelzpunkt von mindestens 39 °C und nicht mehr als 45 °C aufweist.

8. Injektionsvorrichtung, die für die Verwendung in einem Verfahren zum Okkludieren eines Kanals in einem lebenden Säugetier geeignet ist, wobei die Vorrichtung umfaßt:
(a) ein Reservoir, das eine feste, thermoplastische, nichtimmunogene und biokompatible Zusammensetzung umfaßt (14), die ein kristallines thermoplastisches Polymer mit einem Schmelzpunkt von 30 bis 50 °C umfaßt,
(b) eine Injektionsdüse (102), die mit dem Reservoir in Verbindung steht,
(c) Druckmittel (121) zum Ausüben eines Druckes auf die okkludierende Zusammensetzung (14), so daß, wenn die Zusammensetzung geschmolzen ist, sie durch die Injektionsdüse (102) durch Betätigen der Druckmittel extrudiert werden kann, und
(d) einen elektrischen Heizer (12), der, wenn er mit einer geeigneten Energiequelle (131) verbunden ist, die okkludierende Zusammensetzung erhitzen, so daß sie geschmolzen wird.

9. Injektionsvorrichtung nach Anspruch 8, bei der die okkludierende Zusammensetzung eine Zusammensetzung gemäß einem der Ansprüche 1 bis 7 ist.

10. Verwendung eines kristallinen thermoplastischen Polymers mit einem Schmelzpunkt von 34 bis 45 °C bei der Bildung einer thermoplastischen, nicht-immunogenen, biokompatiblen Zusammensetzung zum Okkludieren eines Kanals in einem lebenden Menschen, wobei der Kanal ein Tränenkanal, eine Fallopian-Röhre, ein Vas Deferens, eine Arterie, ein Blutgefäß oder ein Hohlraum in einem Knochen ist.

11. Verwendung nach Anspruch 10, bei der das kristalline Polymer ein Polymer ist, wie es in einem der Ansprüche 3 bis 5 definiert ist.

## Revendications

1. Composition thermoplastique non immunogène et biocompatible qui convient pour une utilisation dans une méthode d'occlusion d'un canal chez un mammifère vivant ; qui est solide à la température du canal et devient fluide lorsqu'elle est chauffée à une température à laquelle elle peut être injectée dans le canal sans endommager les cellules du mammifère; et qui comprend :
(i) un polymère thermoplastique cristallin ayant un point de fusion à l'état cristallin de 30 à 50° C, de préférence de 34 à 45° C, et
(ii) un additif biologiquement inerte qui est opaque aux rayons X.

2. Composition selon la revendication 1 dans laquelle l'additif est de la poudre d'or.

3. Composition selon la revendication 1 ou la revendication 2 dans laquelle le polymère cristallin comprend un polymère cristallin à chaîne latérale.

4. Composition selon la revendication 3 dans laquelle le polymère cristallin est un élastomère thermoplastique.

5. Composition selon la revendication 3 ou 4 dans laquelle le polymère cristallin à chaîne latérale comprend des motifs dérivant d'au moins un acrylate ou méthacrylate d'alkyle dans lequel les groupes alkyle contiennent de 14 à 22 atomes de carbone.

6. Composition selon l'une quelconque des revendications précédentes, qui comprend au moins une substance biologiquement active, en particulier un agent de coagulation du sang, un antibiotique, un spermicide, une hormone de croissance ou un antibiotique.

7. Composition selon l'une quelconque des revendications 1 à 6 convenant pour l'occlusion d'un canal lacrymal chez un être humain, la composition ayant un point de fusion d'au moins 39° C et de pas plus de 45° C.

8. Dispositif d'injection qui convient pour une utilisation dans une méthode d'occlusion d'un canal chez un mammifère vivant, ledit dispositif comprenant :
(a) un réservoir qui contient une composition solide thermoplastique non immunogène et biocompatible (14) comprenant un polymère thermoplastique cristallin ayant un point de fusion de 30 à 50° C
(b) une buse d'injection (102) qui communique avec le réservoir;
(c) des moyens (121) pour exercer une pression sur la composition d'occlusion (14) de sorte que si la composition est fondue elle puisse être extrudée à travers la buse d'injection (102) à l'aide des moyens de mise sous pression; et
(d) un réchauffeur électrique (12) qui, lorsqu'il est connecté à une source d'énergie appropriée (131), chauffera la composition d'occlusion de sorte qu'elle soit à l'état fondu.

9. Dispositif d'injection selon la revendication 8 dans lequel la composition d'occlusion est une composition selon l'une quelconque des revendications 1 à 7.

10. Utilisation d'un polymère thermoplastique cristallin ayant un point de fusion de 34 à 45° C dans la préparation d'une composition thermoplastique non immunogène biocompatible pour l'occlusion d'un canal chez un être humain, le canal étant un canal lacrymal, une trompe de fallope, un canal déférent, une artère, un vaisseau sanguin, ou une cavité dans un os.

11. Utilisation selon la revendication 10 dans laquelle le polymère cristallin est un polymère tel que défini dans l'une quelconque des revendication 3 à 5.
